# EUROPEAN PATENT APPLICATION

(11) **EP 4 527 613 A1**
(43) Date of publication of application: **26.03.2025**
(21) Application number: 23807578.2
(22) Date of filing: 12.05.2023
(51) Int. Cl.: B32B 3/06, A41D 13/00, A41D 13/11, A42B 3/20, A44B 17/00, A44B 18/00, A44B 99/00

(54) **FILM LAMINATE, METHOD FOR PRODUCING FILM LAMINATE, PROTECTOR AND METHOD FOR PRODUCING PROTECTOR**

(30) Priority: 19.05.2022 JP 2022082367
(71) Applicant: Dexerials Corporation, Shimotsuke-shi, Tochigi 323-0194 (JP)
(72) Inventor: KATO, Takuya, Shimotsuke-shi, Tochigi 323-0194 (JP); OISHI, Kiyokazu, Shimotsuke-shi, Tochigi 323-0194 (JP)
(74) Representative: Müller-Boré & Partner Patentanwälte PartG mbB
(86) International application number: PCT/JP2023/017965
(87) International publication number: WO 2023/223976

(57) **Abstract**

A film laminate that can achieve sufficient sterilization effects and does not have the risk of damaging the film surface structure is provided. The film laminate includes a plurality of film-shaped members 2 laminated in a separable manner, and a first engagement member 6 that is fixed to one of a pair of the film-shaped members 2 and a second engagement member 9 that is fixed to the other of the pair of film-shaped members 2, wherein the first engagement member 6 and the second engagement member 9 are releasably engaged with each other to laminate the film-shaped members 2 with a gap therebetween, and the first engagement member 6 and the second engagement member 9 include engagement portions having air permeability.

## Description

### TECHNICAL FIELD

The present technology relates to a film laminate including multiple film-shaped members laminated at a predetermined interval, a method for manufacturing a film laminate, protective equipment using a film laminate, and method for manufacturing protective equipment. This application claims priority based on Japanese Patent Application No. 2022-082367 filed in Japan on May 19, 2022, which is incorporated herein by reference.

### BACKGROUND ART

Personal protective equipment is widely used as a means of preventing occupational infections among medical personnel. In particular, protective equipment such as face shields and protective clothing, which are used to prevent exposure to blood and body fluids, are used during examinations and surgeries and are useful for preventing occupational infections from blood-borne pathogens (HIV, HBV, HCV) and coronaviruses (such as SARS-CoV-2).

However, the surface of this type of protective equipment may become contaminated with blood or body fluids that have splashed off the patient during an examination or surgery. In such an environment where contaminants are flying around, it is necessary to quickly restore visibility while avoiding contact with the contaminants.

In addition, helmet shields used in motorcycle and auto racing, as well as protective eyewear used in painting, have the same problem of frequently dirtying the surface and obstructing visibility.

To solve this problem, it has been conventionally done to laminate several layers of easily separable protective films on the surface of the helmet shield or protective glasses for painting, and when the vision is obstructed by dirt, the topmost protective film is peeled off along with the dirt to restore the vision. With regard to this kind of technology, for example, in Patent Document 1, a protective device for the shield of a helmet is disclosed, which is covered with multiple sheet-shaped protective covers, i.e., disposable visors.

However, if the protective equipment is made as described above, with a simple laminated structure of protective film, reflection occurs at the interface between each of the laminated layers. There are also problems such as a decrease in transmissivity as the number of layers increases.

On the other hand, there is also proposed protective equipment using optical elements with a moth-eye structure with a pitch smaller than the wavelength of visible light and films with low-reflection characteristics to provide excellent visibility (Patent Document 2).

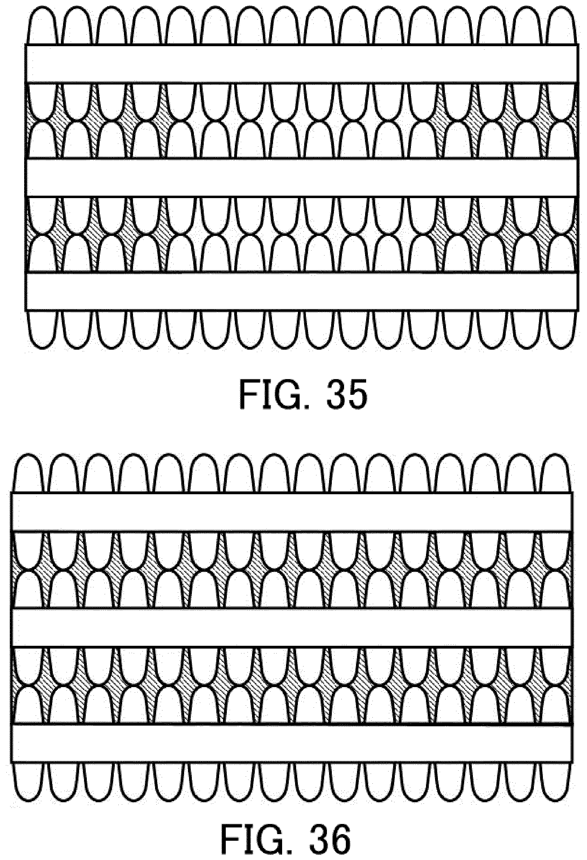

As shown in FIGS. 35 and 36, the laminate described in Patent Document 2 includes multiple film-shaped members having a moth-eye structure consisting of unevenness with a pitch smaller than the visible light wavelength on at least one surface of the base body and is formed by laminating the film-shaped members in a separable manner by fixing them via a pressure-sensitive adhesive layer at least at the edges of the film-shaped members. In the laminate shown in FIG. 35, the pressure-sensitive adhesive layer is provided only at the edges of the film-shaped members. In addition, the laminate shown in FIG. 36 has a pressure-sensitive adhesive layer covering the entire surface of the film-shaped member.

By laminating film-shaped members having the moth-eye structure, it is possible to prevent increases in reflectivity and decreases in transmissivity, thereby ensuring visibility. In addition, when the surface of the laminate becomes contaminated with blood or body fluids, or when dirt obscures the vision, the topmost film-shaped member can be peeled off along with the dirt to restore visibility.

### CITATION LIST

### PATENT LITERATURE

Patent document 1: JP 2000-192322 A
Patent document 2: JP 2015-057317 A

### SUMMARY OF INVENTION

### TECHNICAL PROBLEM

When protective equipment provided with such laminated film-shaped members is used for medical purposes, the surfaces of each film-shaped member are sterilized by gas sterilization or other methods. Then, when the contaminated topmost film-shaped member is peeled off, a new sterile surface is exposed on the outermost surface.

However, in the case of protective equipment that uses a laminate in which the film-shaped members are laminated and fixed together with a pressure-sensitive adhesive layer, when sterilization is carried out using gas or other means, the surfaces of the film-shaped members are not exposed in the areas bonded with the pressure-sensitive adhesive layer, so the sterilizing gas cannot flow in and the unexposed surface is not sterilized.

In the configuration shown in FIG. 35, the central part of the film without the pressure-sensitive adhesive layer can be sterilized, but the outer edge of the film with the pressure-sensitive adhesive layer cannot be sterilized. Furthermore, in the configuration shown in FIG. 35, if the pressure-sensitive adhesive layer covers the entire circumference of the film, the front surface and the back surface of the film enclosed by the pressure-sensitive adhesive layer will not be sterilized. In the configuration shown in FIG. 36, only the topmost surface and the bottom surface of the laminate are sterilized, and the front surface and the back surface of the film with the pressure-sensitive adhesive layer in between cannot be sterilized.

Therefore, when the contaminated topmost film-shaped member is peeled off, a film-shaped member that has not been sterilized in some areas or has not been sterilized at all is exposed; which means that the sterilization process was not fully effective.

In the case of a transparent laminate with a moth-eye structure formed on the film surface laminated using a pressure-sensitive adhesive, there is a risk that the moth-eye structure may be destroyed when peeling off the individual film-shaped member, depending on the pressure-sensitive adhesive strength.

Therefore, an object of the present technology is to provide a film laminate capable of achieving sufficient sterilization effects without the risk of damaging the film surface structure, as well as a method for manufacturing a film laminate, protective equipment, and a method for manufacturing protective equipment.

### SOLUTION TO PROBLEM

In order to solve the problem, a film laminate according to the present technology includes a plurality of film-shaped members laminated in a separable manner, and a first engagement member that is fixed to one of a pair of the film-shaped members and a second engagement member that is fixed to the other of the pair of film-shaped members, wherein the first and second engagement members are releasably engaged with each other to laminate the film-shaped members with a gap therebetween, and the first and second engagement members include engagement portions having air permeability.

A method for manufacturing a film laminate according to the present technology includes a step of forming film-shaped members, a step of forming a first engagement member and a second engagement member that are releasably engaged with each other, a step of fixing the first engagement member to one film-shaped member of a pair of film-shaped members at a position facing the other film-shaped member of the pair of film-shaped members, and fixing the second engagement member to the other film-shaped member of the pair of film-shaped members at a position facing the one film-shaped member of the pair of film-shaped members, and a step of laminating the pair of film-shaped members with a gap therebetween by engaging the first and second engagement members.

In addition, protective equipment according to the present technology is protective equipment in which a film laminate is attached to a part corresponding to the face or eyes of a user, wherein the film laminate is as described above.

In addition, a method for manufacturing protective equipment according to the present technology is a method for manufacturing protective equipment in which a film laminate is attached to a part corresponding to the face or eyes of a user, including a step of forming a film laminate, and a step of attaching the film laminate to protective equipment, wherein the film laminate is as described above.

### ADVANTAGEOUS EFFECTS OF INVENTION

According to this technology, it is possible to expose the entire front and back surfaces of each film-shaped member of the film laminate, including the first and second engagement members, to a sterilizing gas, and when a film-shaped member is peeled off, a newly revealed film-shaped member and the first or second engagement members fixed to it have also been sterilized. In addition, the film-shaped member can be peeled off simply by releasing the engagement between the first and second engagement members, so there is no risk of damaging the moth-eye structure formed on the film-shaped member.

### BRIEF DESCRIPTION OF DRAWINGS

FIG. 1 is a disassembled perspective view of a film laminate according to the present technology.
FIG. 2 is a cross-sectional view of the film laminate according to the present technology.
FIG. 3 is a cross-sectional view illustrating a loop member.
FIG. 4 is a cross-sectional view illustrating a hook member.
FIG. 5 is a schematic view illustrating a configuration in which the loop member and the hook member are fixed to the left and right side edges of roughly rectangular film-shaped members in a line pattern.
FIG. 6 is a schematic view illustrating a configuration in which the loop member and the hook member are fixed to the left and right side edges of roughly rectangular film-shaped members in a dashed line pattern.
FIG. 7 is a schematic view illustrating a configuration in which the loop member and the hook member are fixed to the top and bottom side edges of roughly rectangular film-shaped members in a line pattern.
FIG. 8 is a schematic view illustrating a configuration in which the loop member and the hook member are fixed to the top and bottom side edges of roughly rectangular film-shaped members in a dashed line pattern.
FIG. 9 is a schematic view illustrating a configuration in which the loop member and the hook member are fixed to surround the entire circumference of the side edges of roughly rectangular film-shaped members.
FIG. 10 is a schematic cross-sectional view illustrating a configuration in which the width of the loop and hook members to laminate the topmost film-shaped member and the intermediate film-shaped member is narrower than the width of the loop and hook members to laminate the intermediate film-shaped member and the bottommost film-shaped member, thereby reducing the relative peel strength.
FIG. 11 is a cross-sectional view of a film-shaped member constituting a film laminate according to the present technology.
FIG. 12 is a cross-sectional view of another film-shaped member constituting a film laminate according to the present technology.
FIG. 13 is a perspective view of another film-shaped member constituting a film laminate according to the present technology.
FIG. 14 is a perspective view of a roll master for transfer printing, on which a pattern of anti-reflection layer composed of a moth-eye structure has been formed.

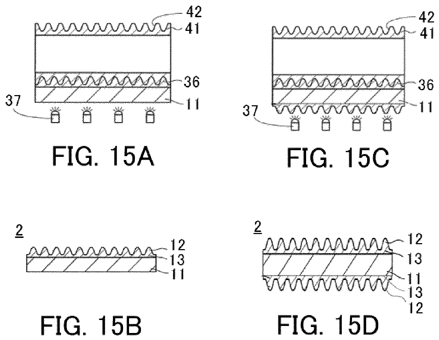

FIG. 15 shows cross-sectional views illustrating the steps of forming an anti-reflection layer composed of a moth-eye structure, in which FIG. 15A shows a state wherein a side of the base material to which the transfer material has been applied is brought into close contact with the roll master, FIG. 15B shows a film-shaped member on which the moth-eye structure has been transferred to one side, FIG. 15C shows a state wherein the transfer material is applied to the other side of the film-shaped member other than the side to which the moth-eye structure has been transferred, and the other side is brought into close contact with the roll master, and FIG. 15D shows the film-shaped member with the moth-eye structure transferred to both sides.
FIG. 16 is an exterior perspective view illustrating a protective suit for medical use as an example of protective equipment.
FIG. 17 is a cross-sectional view illustrating an example of a step of attaching a film laminate to protective equipment, in which FIG. 17A shows the state before the attachment and FIG. 17B shows the state after the attachment.
FIG. 18 is a cross-sectional view illustrating an example of a step of attaching a film laminate to protective equipment using a loop member and a hook member instead of an adhesive layer.
FIG. 19 is a cross-sectional view illustrating another example of a step of attaching a film laminate to protective equipment.
FIG. 20 is a cross-sectional view illustrating another example of a step of attaching a film laminate to protective equipment using a loop member and a hook member instead of an adhesive layer.
FIG. 21 is a cross-sectional view illustrating an example of a step of forming a film laminate and attaching it to protective equipment, in which FIG. 21A shows the state where the bottommost film-shaped member is connected to an opening of protective equipment, FIG. 21B shows the state where the intermediate film-shaped member is laminated, and FIG. 21C shows the state where the topmost film-shaped member is laminated.
FIG. 22 is a cross-sectional view illustrating another configuration example of a film laminate according to the present technology, in which FIG. 22A shows the state before the film-shaped members are laminated, and FIG. 22B shows the state after the film-shaped members are laminated.
FIG. 23 is a cross-sectional view illustrating the step of attaching the film laminate shown in FIG. 22 to protective equipment, in which FIG. 23A shows the state before the film laminate is attached, and FIG. 23B shows the state after the film laminate is attached.
FIG. 24 is a cross-sectional view illustrating an example of a snap button, which is an example of the configuration of the first and second engagement members.
FIG. 25 shows another example of the configuration of the recessed member and the protruding member, in which FIG. 25A is a disassembled perspective view of a recessed member and a protruding member, FIG. 25B is a perspective view illustrating the step of inserting a protruding portion of a substrate into a hole of a film-shaped member, FIG. 25C is a perspective view illustrating the step of fitting the lid of the recessed member to the protruding portion, and FIG. 25D is a perspective view illustrating the step of fitting the lid of the protruding member to the protruding portion.
FIG. 26 shows a film laminate made by laminating film-shaped members using the recessed member and the protruding member shown in FIG. 25, in which FIG. 26A is a side view illustrating the step of laminating a film-shaped member with the recessed member fixed thereon and a film-shaped member with the protruding member fixed thereon, FIG. 26B is a side view illustrating a film laminate consisting of two layers of film-shaped members, and FIG. 26C is a side view illustrating a film laminate consisting of three layers of film-shaped members.
FIG. 27 is a plan view illustrating an example of a zipper that is an example of the configuration of the first and second engagement members.
FIG. 28 is an explanatory view of the configuration of the film laminate sample for the first example.
FIG. 29 is a perspective view illustrating a method for measuring the peel strength.
FIG. 30 is an explanatory view of the structure of the film laminate sample for the first embodiment, in which FIG. 30A shows an example using a loop member and a hook member of the same size as the film-shaped member, and FIG. 30B shows an example using a loop member and a hook member with a base material size reduced by half.
FIG. 31 is an explanatory view of the structure of a film laminate sample in which the loop member and the hook member are provided at both ends of the film-shaped member in the longitudinal direction.
FIG. 32 is an explanatory view of the structure of a film laminate sample in which the loop member and the hook member are provided to surround all four sides of the film-shaped member.
FIG. 33 is a photograph showing the results of a sterilization test on a film laminate sample in which the loop member and the hook member are provided to surround all four sides of the film-shaped member.
FIG. 34 is a photograph showing the results of a sterilization test on a film laminate sample in which an adhesive is provided to surround all four sides of the film-shaped member.
FIG. 35 is a cross-sectional view of a laminate in which multiple film-shaped members are laminated using pressure-sensitive adhesive layers provided on the edges of the film-shaped member.
FIG. 36 is a cross-sectional view of a laminate in which multiple film-shaped members are laminated together using pressure-sensitive adhesive layers provided on the entire surfaces of the film-shaped members.

### DESCRIPTION OF EMBODIMENTS

Embodiments of a film laminate, a manufacturing method for a film laminate, protective equipment, and a manufacturing method for protective equipment according to the present technology will now be more particularly described with reference to the accompanying drawings. It should be noted that the present technology is not limited to the embodiments described below and various modifications can be added to the embodiment without departing from the scope of the present technology. The features shown in the drawings are illustrated schematically and are not intended to be drawn to scale. Actual dimensions should be determined in consideration of the following description. Moreover, those skilled in the art will appreciate that dimensional relations and proportions may be different among the drawings in some parts.

### FILM LAMINATE

A film laminate 1 according to the present technology includes a plurality of film-shaped members 2 laminated in a separable manner, and a first engagement member that is fixed to one film-shaped member 2 of a pair of film-shaped members 2 that are laminated adjacent to each other, and a second engagement member that is fixed to the other film-shaped member 2 that faces the one film-shaped member. The film-shaped member 2 is a flexible transparent sheet. The first engagement member and the second engagement member are releasably engaged with each other. As a result, the pair of film-shaped members 2 are laminated next to each other with a gap therebetween. The first and second engagement members are partially engaged without being in close contact, and the engagement portion has a gap and air permeability. In other words, the film laminate 1 is formed by laminating a plurality of film-shaped members 2 with a gap therebetween, and the engagement portions of the first and second engagement members of each film-shaped member 2 are not sealed.

Therefore, the film laminate 1 makes it possible to expose the entire front and back surfaces of each film-shaped member 2, including the first and second engagement members, to the sterilizing gas, and when the film-shaped member 2 is peeled off, the newly revealed film-shaped member 2 and the first or second engagement member fixed to it have also been sterilized.

In medical applications, if the surface becomes contaminated with blood or body fluids splashed from the patient during an examination or surgery, the topmost film-shaped member 2 can be peeled off to quickly restore visibility while avoiding contact with the contaminant. At this time, since the entire surface of the film-shaped member 2 exposed by peeling off the film laminate 1 and the first and second engagement members have been sterilized, the risk of infection to the patient, the examinee, and the medical personnel can be reduced. In addition, since the back surface of the peeled-off film-shaped member 2 has also been sterilized as the same manner, there is no risk of exposing the patient, examinee, and medical personnel to unsterilized areas when the film-shaped member 2 is peeled off or disposed of.

### FIRST AND SECOND ENGAGEMENT MEMBER

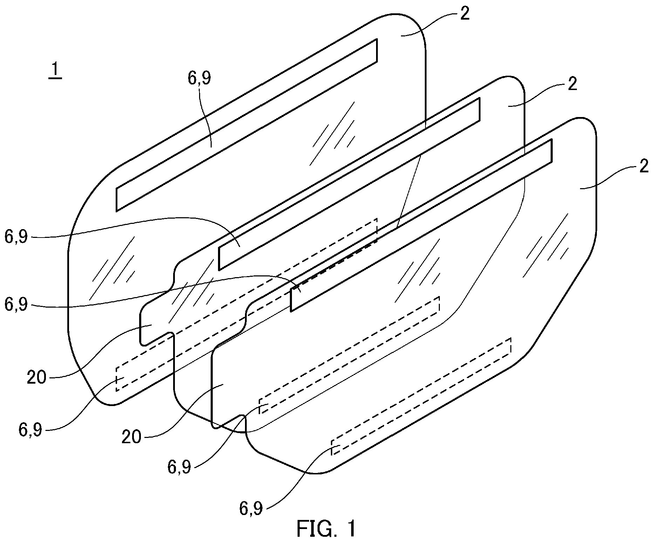

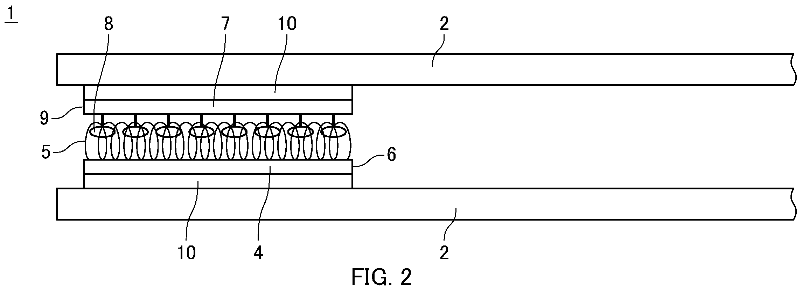

The film laminate 1 shown in FIGS. 1 and 2 employs a loop member 6 with a number of loops 5 erected on one surface of a base material 4 as the first engagement member, and a hook member 9 with a number of hooks 8 erected on one surface of a base material 7 that can be releasably engaged with the above loops 5 as the second engagement member.

The film-shaped member 2 has one of the loop member 6 and the hook member 9 on one side, and the intermediate film-shaped member 2 has the other of the loop member 6 and the hook member 9 on the other side. For convenience, the figures in this application are labeled "6, 9" to indicate that one or the other of the loop member 6 and the hook member 9 is provided on one side and/or the other side.

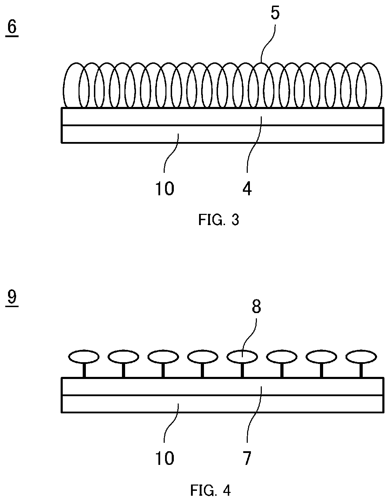

As shown in FIG. 3, the base material 4 of the loop member 6 is a sheet-shaped support for the loops 5, and a number of minute loops 5 are erected on one side, and the other side opposite the one side becomes a fixing surface that is fixed to the film-shaped member 2.

As shown in FIG. 4, the base material 7 of the hook member 9 is a sheet-shaped support for the hooks 5, and a number of minute hooks 8 are erected on one side, and the other side opposite the one side is a fixing surface that is fixed to the film-shaped member 2. The hook 5 has a shape that can engage with the loop 5, e.g. J-shaped or mushroom-shaped, but this is not limited to these shapes.

In the film laminate 1, the base material 4 of the loop member 6 is fixed to one of the film-shaped members 2 in a pair of adjacent film-shaped members 2 using a fixing material 10 such as an adhesive or pressure-sensitive adhesive tape, and the base material 7 of the hook member 9 is adhered to the other film-shaped member 2 facing the one film-shaped member 2 using the fixing material 10. In addition, the loop member 6 and the hook member 9 are provided in positions facing each other when the film-shaped members 2 are laminated.

By stacking the film-shaped member 2 to which the loop member 6 is fixed with the film-shaped member 2 to which the hook member 9 is fixed, the hooks 8 engage with the loops 5. As a result, one film-shaped member 2 and the other film-shaped member 2 are held and laminated with a gap therebetween, thereby enabling sterilization treatment with sterilizing gas. In addition, even if there is no apparent gap between one film-shaped member 2 and the other film-shaped member 2 when viewed from the side, these films are not in close contact with each other but are only slightly touching, and the sterilizing gas is able to pass through the layers.

In addition, the loop member 6 and the hook member 9 are partially engaged with the loop 5 and hook 8 that are erected on the base material 4, 7 without the base material 4, 7 adhering to each other, and there is a gap between the loop member 6 and the hook member 9, so it has air permeability. Therefore, the film laminate 1 can allow the sterilizing gas to pass through when the loop member 6 and the hook member 9 are engaged, i.e. when the film-shaped member 2 is laminated. Therefore, in the film laminate 1, even when the loop member 6 and the hook member 9 are fixed so as to surround the outer edge of the film-shaped member 2, the surface of the film-shaped member 2 surrounded by the loop member 6 and the hook member 9 can be sterilized.

The film-shaped member 2 is peeled off by releasing the engagement of the loop member 6 and the hook member 9. This exposes the sterilized surface of the next film-shaped member 2. In addition, the engagement portions of the loop member 6 and the hook member 9, which are permeable, are also sterilized, so the sterilized loop member 6 and the hook member 9 are exposed.

As such loop member 6 and hook member 9, e.g. 3M mechanical fasteners or so-called hook and loop fasteners can be used.

### FIXING POSITION

The loop member 6 and the hook member 9 are provided in such a position and number that the film-shaped member 2 can be stably held and laminated with a gap therebetween, and that the user's field of view within the film-shaped member 2 is not obstructed in use, and that easy peeling is possible. From this perspective, it is preferable that the loop member 6 and the hook member 9 are formed into tape shapes and are attached to the side edges of the film-shaped member 2.

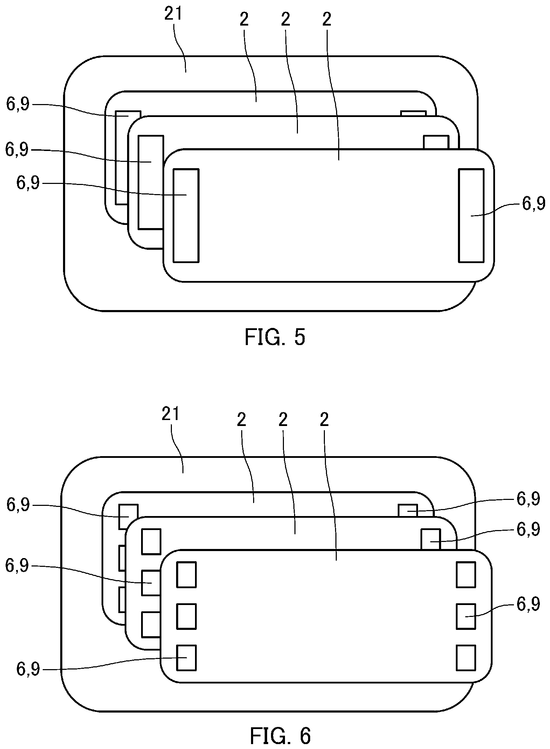

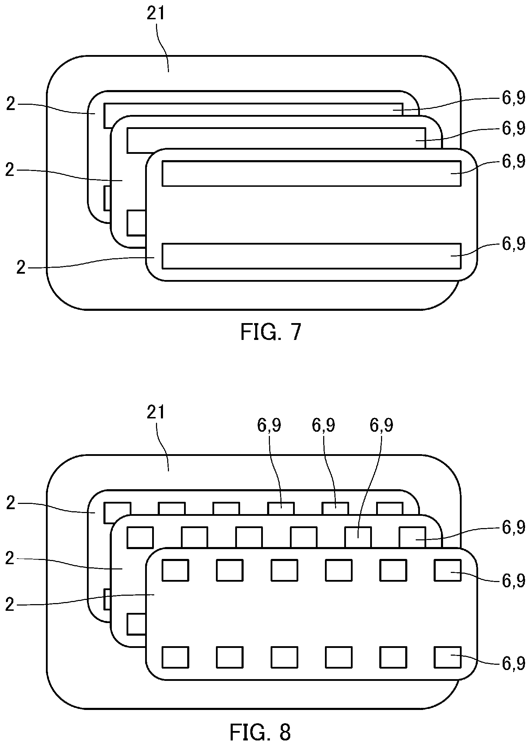

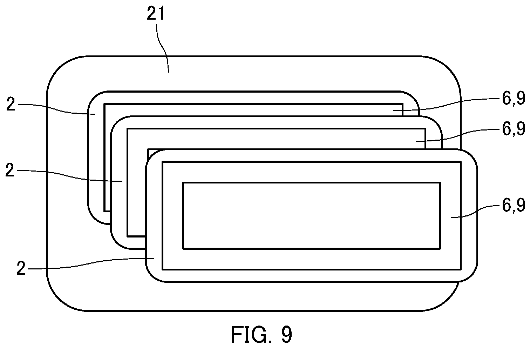

The attachment pattern can be determined according to the shape and purpose of use of the protective equipment to be attached. For example, the loop member and the hook member may be fixed to the left and right side edges of roughly rectangular film-shaped members 2 in a line pattern as shown in FIG. 5, to the left and right side edges of roughly rectangular film-shaped members 2 in a dashed line pattern as shown in FIG. 6, to the top and bottom side edges of roughly rectangular film-shaped members 2 in a line pattern as shown in FIG. 7, or to the top and bottom side edges of roughly rectangular film-shaped members 2 in a dashed line pattern as shown in FIG. 8. Alternatively, as shown in FIG. 9, the loop member and the hook member may be fixed to surround the entire circumference of the side edges of the film-shaped member 2. FIGS. 5 to 9 are schematic views illustrating the fixing patterns of the loop member 6 and the hook member 9, which are used to mutually laminate the film-shaped members 2. The loop member 6 or the hook member 9 is fixed to one or both of the front and back surfaces of the film-shaped member 2, depending on the lamination position.

In addition, a plurality of loop members 6 and hook members 9 may be fixed in parallel to the side edges of the film-shaped member 2. The positions and number of the loop members 6 and the hook members 9 are not limited to those described above and are set as appropriate according to the configuration of the protective equipment 21 and the film laminate 1, and its application, among other factors.

In addition, the peel strength required to separate each tape-shaped loop member 6 and hook member 9 may be the same or different. For example, when fixing the tape-shaped loop member 6 and hook member 9 in a dashed line pattern, the loop member 6 and the hook member 9 fixed near the corner of the film-shaped member 2 can be made to have a relatively strong peel strength compared to those fixed in the middle, or when fixing the tape-shaped loop member 6 and hook member 9 to the top and bottom side edges, it is also possible to vary the peel strength for each part, such as by fixing a relatively wide loop member 6 and hook member 9 to the top edge and a relatively narrow loop member 6 and hook member 9 to the bottom edge. In addition, the peel strength may be made different for each part by changing the hook shape or the width of a single tape-shaped loop member 6 and hook member 9.

Furthermore, tape-shaped loop members 6 and hook members 9 may be mixed on one side of the film-shaped member 2. In this case, the tape-shaped loop members 6 and hook members 9 are also mixed on the opposing film-shaped member 2, and arranged so that they face each other.

### PEEL STRENGTH

In the film laminate 1, it is preferable that the peel strength of the loop member 6 and the hook member 9 to laminate a relatively upper layer of the film-shaped members 2 be lower than the peel strength of the loop member 6 and the hook member 9 to laminate a relatively lower layer of the film-shaped members 2. This prevents the loop member 6 and the hook member 9 that are holding and laminating the relatively lower-layered film-shaped members 2 from being released when the upper-layered film-shaped members 2 are peeled off and prevents the lower-layered film-shaped members 2 from being peeled off together with the upper-layered film-shaped members 2.

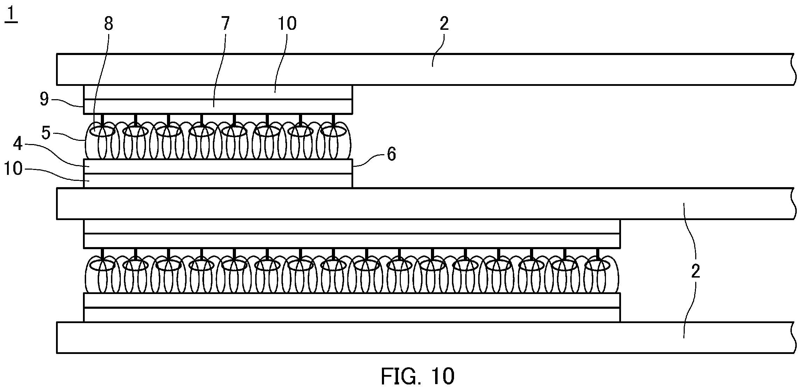

The peel strength between the film-shaped members 2 can be adjusted by changing the hook shape of the loop member 6 and the hook member 9 provided between each film-shaped member 2, changing the number of fixed loops and hooks of the loop member 6 and the hook member 9, or changing the width of the loop member 6 and the hook member 9 to change the engagement area. FIG. 10 is a schematic cross-sectional view illustrating a configuration in which the width of the loop member 6 and the hook member 9 to laminate the topmost film-shaped member 2 and the intermediate film-shaped member 2 is narrower than the width of the loop member 6 and the hook member 9 to laminate the intermediate film-shaped member 2 and the bottommost film-shaped member 2, thereby reducing the relative peel strength. The film laminate 1 shown in FIG. 10 can prevent the intermediate film-shaped member 2 from being peeled off when the topmost film-shaped member 2 is peeled off.

In addition, the fixing strength of the fixing material 10 that fixes the loop member 6 and the hook member 9 to the film-shaped member 2 is higher than the peel strength between each film-shaped member 2 of the film laminate 1. Therefore, when the film-shaped member 2 is peeled off, the loop member 6 and the hook member 9 will not be peeled off at the interface with the film-shaped member 2. Also, when the film-shaped member 2 is peeled off, the fixing material 10 itself will not cause a cohesion failure between the fixed layers.

### OTHERS

In the above, the loop member 6 and the hook member 9 were used as the first and second engagement members that hold and laminate the film-shaped members 2, but the first engagement member may be changed to the hook member 9 if it has the above-mentioned action and effect. In other words, the film-shaped member 2 may be held and laminated by engaging the hook members 9 with each other.

### FILM-SHAPED MEMBER

The film-shaped member 2 is a flexible transparent sheet. There is no particular restriction on the shape of the film-shaped member 2, and it can be selected as appropriate according to the protective equipment 21 to be applied, e.g., as a roughly rectangular shape as shown in FIG. 1.

As mentioned above, the loop members 6 or the hook members 9 are provided on the side edges of the film-shaped member 2. Specifically, the topmost film-shaped member 2 has the loop members 6 or the hook members 9 provided on the back surface, and the intermediate film-shaped member 2 has the loop members 6 or the hook members 9 provided on the front surface and back surface. The bottommost film-shaped member 2 has the loop members 6 or the hook members 9 on the front surface, and the loop member 6 or the hook member 9 are provided on the back surface as appropriate according to the mounting form with the protective equipment 21.

The film-shaped member 2 may have a tab 20 for peeling at the outer edge. The tab 20 is a part that is pinched to peel off the film-shaped member 2. It is preferable to give the tab 20 a function to identify the film-shaped member 2 to be peeled off. This makes it possible to prompt the user to peel off the topmost layer film-shaped member 2 and prevents the user from accidentally peeling off the intermediate film-shaped member 2 together with the topmost film-shaped member 2.

One way to identify the topmost film-shaped member 2 is to make the tabs 20 successively smaller from the topmost layer. In other words, as shown in FIG. 1, the tab 20 formed on the topmost film-shaped member 2 is made larger than the tabs 20 on the film-shaped members 2 below it to hide the tabs on the lower film-shaped members 2, so that it is always easy to pinch only the tab 20 on the topmost film-shaped member 2, and it is prevented from accidentally pinching the tabs 20 on the lower film-shaped members 2.

In addition, the position of the tab 20 may be changed for each film-shaped member 2. For example, the topmost film-shaped member 2 has a tab 20 on the right side edge in a front view, and the intermediate film-shaped member 2 has a tab 20 on the left side edge in a front view. In this way, by first pinching the tab 20 on the right side edge, the topmost film-shaped member 2 can be peeled off, and it is possible to prevent the intermediate film-shaped member 2 from being peeled off by mistake.

In addition, it is also possible to identify the film-shaped members 2 by changing the color of the tab 20 for each film-shaped member 2, or by processing physical features such as uneven marks, openings, and notches. In addition, the above identification means may be combined.

Furthermore, the film laminate 1 may have all the same film-shaped members 2 for each layer, or it may have film-shaped members 2 with different functions or optical characteristics. These configurations are selected as appropriate according to the application of the protective equipment 21 to which the film laminate 1 is applied. In addition, the laminating order of the film-shaped members 2 with different functions or characteristics is also set as appropriate according to the application of the protective equipment 21.

### MOTH-EYE STRUCTURE

The film-shaped member 2 is preferably an optical element with an anti-reflection function, in which multiple structures are provided on at least one surface of a flexible transparent base material at a pitch smaller than the wavelength of visible light. The fine uneven structure with an anti-reflection function will be referred to as the "moth-eye structure" in the following. Furthermore, in the film laminate 1, by using a film-shaped member 2 with a moth-eye structure, the film-shaped member 2 can be laminated without impairing visibility.

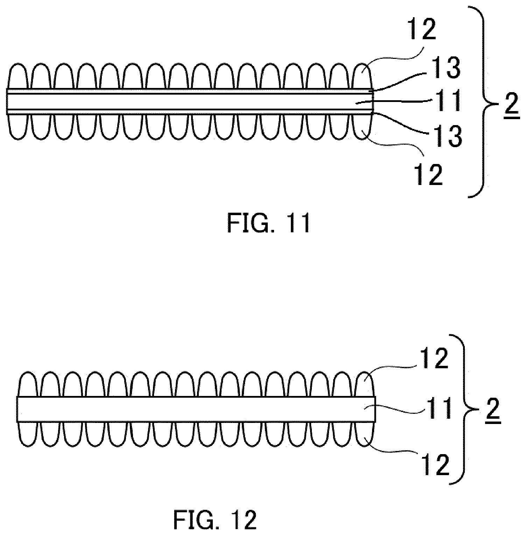

As shown in FIG. 11, the film-shaped member 2 has structures 12 with a pitch smaller than the wavelength of visible light on both sides of a base body 11 via a base layer 13 and thereby has an anti-reflection function on both the front surface and the back surface opposite to each other. The multiple structures 12 are regularly arranged in multiple rows on the front surface and the back surface of the base body 11, on top of the base layer 13. In other words, the front surface and the back surface of the film-shaped member 2 have unevenness made up of the multiple structures 12, i.e., the moth-eye structure. The film-shaped member 2 may have the structures 12 only on the front surface of the base body 11.

By providing such unevenness on the front surface and the back surface of the film-shaped member 2, the protective equipment 21 to which the film laminate 1 is attached will have an excellent optical adjustment function with little wavelength dependence and excellent visibility. In other words, this can contribute to achieving protective equipment 21 with excellent visibility.

Here, "optical adjustment function" refers to adjustment functions for optical characteristics such as transmission characteristics and reflection characteristics. The film-shaped member 2, as an optical element, is transparent to visible light, and the refractive index n thereof is preferably 1.30 to 2.00, and more preferably 1.34 to 2.00. However, the refractive index n is not limited to this range.

In addition, the refractive index of the structures 12 is preferably the same as or approximately the same as the refractive index of the base body 11. This is because the same or approximately the same refractive index will suppress internal reflection and improve contrast.

In FIG. 11, an example is shown in which the structures 12 are formed on the front surface and the back surface of the base body 11 via the base layer 13, and this base layer 13 plays a role in improving the adhesion of the structures 12 to the base body 11. In this case, the base layer 13 is a transparent optical layer integrally molded with the structures 12 on the bottom side of the structures 12 and may be formed by curing an energy ray-curable resin composition, or the like, similar to the structures 12.

In addition, as shown in FIG. 12, the film laminate 1 may use the film-shaped member 2 in which the moth-eye structure of the plurality of structures 12 are directly formed on the base body 11 without the base layer 13.

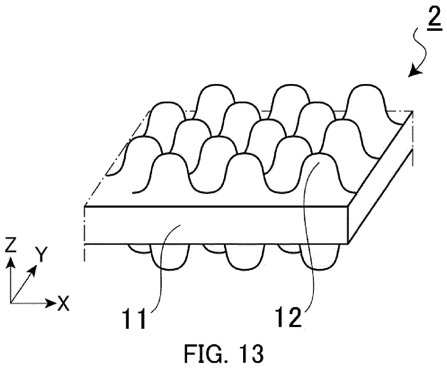

Furthermore, as shown in FIG. 13, the film laminate 1 may use the film-shaped member 2 in which the base body and the structures are integrally molded. The film-shaped member 2 shown in FIG. 13 has the structures 12 integrally molded on both sides of the base body 11.

### BASE BODY

The base body 11 will be further explained here. The base body 11 is composed of, e.g., a transparent base material. For example, the base body 11 is mainly composed of a transparent plastic material but is not limited to these materials.

When using a plastic material as the base body 11, in order to further improve the surface energy, coating properties, slipperiness, and flatness, among other properties, of the plastic material surface, a further undercoating layer not illustrated may be provided by surface treatment. This undercoating layer may include, e.g., organoalkoxymetal compounds, polyesters, acrylic-modified polyesters, and polyurethanes, among others. In addition, in order to obtain the same effect as that of the undercoating layer, it is also possible to carry out corona discharge treatment, UV irradiation treatment, and the like on the surface of the base body 11.

When the base body 11 is a plastic film, the base body 11 can be obtained by, e.g., stretching the above-mentioned resin or dissolving the resin in a solvent and then drying the solution to form a film. The thickness of the base body 11 is preferably selected as appropriate for the application of the film-shaped member 2, and may be, e.g., 10 µm or more and 500 µm or less. The shape of the base body 11 may be, e.g., film-shaped or plate-shaped, but is not limited to these shapes. Note that the term "film" includes sheets.

The examples of the material for the base body 11 may include methyl methacrylate (co) polymer, polycarbonate, styrene (co) polymer, methyl methacrylate-styrene copolymer, cellulose diacetate, cellulose triacetate, cellulose acetate butyrate, polyester, polyamide, polyimide, polyethersulfone, polysulfone, polypropylene, polymethylpentene, polyvinyl chloride, polyvinyl acetal, polyetherketone, polyurethane, and glass, but the material is not limited to these.

### STRUCTURE

Next, the structures 12 will be explained. Generally, the wavelength band of visible light is 360 nm to 830 nm, but in this embodiment, the structures 12 are arranged in a regular pattern with a size less than the wavelength band of visible light. From this perspective, the arrangement pitch of the structures 12 shall not exceed 350 nm. The structures 12 may be of various shapes, such as conical, columnar, or needle-shaped.

As described below, the structures 12 are formed by using a roll master exposure device in which a pattern corresponding to the moth-eye structure is formed to transfer the pattern to the transfer material 36, such as an energy ray-curable resin composition coated on the base body 11 and then curing the transfer material 36.

The cured material of the transfer material 36 may have hydrophilic properties. It is preferable that the transfer material 36 contains one or more functional groups having hydrophilic properties. Such functional groups having hydrophilic properties include, e.g., hydroxyl groups, carboxyl groups, and carbonyl groups.

In addition, the energy ray-curable resin product that forms the structures 12 may have different physical properties on both sides of the base body 11. For example, depending on the application, the hydrophobic and hydrophilic properties can be used separately to provide specific surfaces with functions such as anti-fogging.

It is preferable to use a UV ray-curable resin composition as the energy ray-curable resin composition. In addition, the energy ray-curable resin composition may contain fillers or functional additives as needed.

The UV ray-curable resin composition contains, e.g., an acrylate and an initiator.

The UV ray-curable resin composition includes, e.g., monofunctional monomers, difunctional monomers, and polyfunctional monomers, and specifically, it is a mixture of the following materials alone or in combination.

Example of the "monofunctional monomer" may include carboxylic acids (acrylic acid), hydroxy groups (2-hydroxyethyl acrylate, 2-hydroxypropyl acrylate, 4-hydroxybutyl acrylate),alkyl or alicyclic monomers (isobutyl acrylate, t-butyl acrylate, iso-octyl acrylates, lauryl acrylate, stearyl acrylate, isobornyl acrylate, cyclohexyl acrylate), and other functional monomers (2-methoxyethyl acrylate, methoxyethylene glycol acrylate, 2-ethoxyethyl acrylate, tetrahydrofurfuryl acrylate, benzyl acrylate, ethyl carbithol acrylate, phenoxyethyl acrylate, N,N-dimethylaminoethyl acrylate, N,N-dimethylaminopropyl acrylamide, N,N-dimethylacrylamide, acryloyl morpholine, N-isopropyl acrylamide, N,N-diethylacrylamide, N-vinylpyrrolidone, 2-(perfluorooctyl)ethyl acrylate, 3-perfluorohexyl-2-hydroxypropyl acrylate, 3-perfluorooctyl-2-hydroxypropyl acrylate, 2-(perfluorodecyl) ethyl acrylate, 2-(perfluoro-3-methylbutyl)ethyl acrylate), 2,4,6-tribromophenol acrylate, 2,4,6-tribromophenol methacrylate, 2-(2,4,6-tribromophenoxy)ethyl acrylate), and 2-ethylhexyl acrylate.

Examples of the "difunctional monomer" may include tri(propylene glycol) diacrylate, trimethylolpropane-diaryl ether, and urethane acrylate.

Examples of the "polyfunctional monomer" may include trimethylolpropane triacrylate, dipentaerythritol penta- and hexa-acrylates, and ditrimethylolpropane tetraacrylate.

Among these, the preferred resin compositions to compose the transfer material 36 include 2-hydroxyethyl acrylate, acrylamorpholine, glycerol acrylate, polyether acrylate, N-vinylformamide, N-vinylpyrrolidone, N-vinylcaprolactone, ethoxydiethylene glycol acrylate, methoxy triethylene glycol acrylate, polyethylene glycol acrylate, EO-modified trimethylolpropane triacrylate, EO-modified bisphenol A diacrylate, aliphatic urethane oligomer, and polyester oligomer.

Examples of the "initiator" may include 2,2-dimethoxy-1,2-diphenylethan-1-one, 1-hydroxy-cyclohexylphenyl ketone, and 2-hydroxy-2-methyl-1-phenylpropan-1-one.

Examples of the "filler" may include both inorganic and organic fine particles. Inorganic fine particles may include metal oxide fine particles such as SiO₂, TiO₂, ZrO₂, SnO₂, and Al₂O₃.

Examples of the "functional additive" may include leveling agents, surface modifiers, and defoaming agents.

By forming the moth-eye structure composed of fine uneven structures as structures 12, the film laminate 1 achieves a high level of anti-reflection function. Here, the anti-reflection performance of the combination of the front surface and the back surface of the film laminate 1 is 5% or less, preferably 1% or less, and even more preferably 0.5% or less. The light source for surgery has an illuminance of 100,000 1x or more, and even a few percent of reflected light can be blinding, so it is necessary to suppress reflection as much as possible. Furthermore, the film laminate 1 can be well used as a face shield, an eye shield, and protective clothing for medical use, because the film laminate 1 having a layer of an anti-reflection film composed of a moth-eye structure having multiple structures with a pitch smaller than the wavelength of visible light as structures 12 on a transparent base body 11, has a low wavelength dependence, a low angle dependence, and a high anti-reflection performance.

In addition, the film laminate 1 can be made to have anti-fogging properties by using a hydrophilic resin to form the multiple structures having a pitch smaller than the wavelength of visible light which constitute the anti-reflection layer composed of a moth-eye structure.

Furthermore, the film laminate 1 can have even better anti-reflection performance by forming an anti-reflection layer composed of a moth-eye structure formed on both sides of the transparent base body 11.

### MANUFACTURING STEPS OF FILM-SHAPED MEMBERS

Next, the steps for manufacturing the film-shaped members 2 with a moth-eye structure formed thereon will be explained. The moth-eye structure is formed by using a roll master exposure device in which a pattern corresponding to the moth-eye structure is formed to transfer the pattern.

### ROLL MASTER

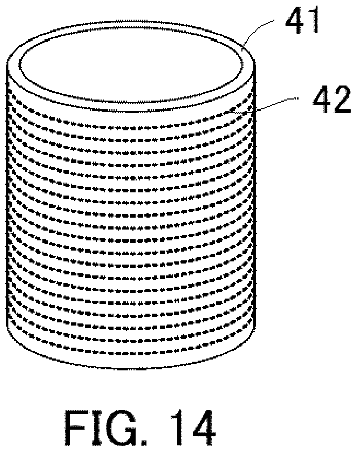

As shown in FIG. 14, a roll master 41 has a round columnar or cylindrical shape, and the round columnar or cylindrical surface is used as the forming surface for forming the plurality of structures 12 on the base body surface. On this forming surface, e.g., predetermined structures 42 are provided in a two-dimensional array by dry etching, wet etching, or the like. The structures 42 have a concave or convex shape with respect to the forming surface. The material of the roll master 41 can be glass, e.g., but is not limited to this material.

The multiple structures 42 arranged on the forming surface of the roll master 41 and the multiple structures 12 arranged on the surface of the base body 11 described above are in an inverted concave-convex relationship. In other words, the shape, arrangement, and pitch of the structures 42 on the roll master 41 are the same as those of the structures 12 on the base body 11.

### TRANSFER STEP 1

As shown in FIG. 15A, after applying the transfer material 36 to one side of the base body 11, the side to which the transfer material 36 is applied is brought into close contact with the roll master 41 in which a pattern corresponding to the moth-eye structure is formed. Next, the transfer material 36 is cured by irradiating it with an energy ray such as an ultraviolet ray from an energy ray source 37, and then the base body 11, which is integrated with the cured transfer material 36, is peeled off. This results in the film-shaped member 2 having multiple structures 12 formed on one side of the base body 11, as shown in FIG. 15B. If necessary, the base layer 13 may be formed between the structures 12 and the base body 11.

The energy ray source 37 may be any source capable of emitting energy rays, such as electron rays, ultraviolet rays, infrared rays, laser rays, visible rays, ionizing radiation rays (such as X-rays, alpha rays, beta rays, gamma rays), microwaves, or radio waves, and is not limited to any particular type.

### TRANSFER STEP 2

When the film-shaped member 2 with multiple structures 12 formed on both sides of a base body 11 is required, as shown in FIG. 15C, after bringing the roll master 41 for the moth-eye structure and the transfer material 36 applied to the surface of the base body 11 opposite to the surface on which the structures are formed into close contact, the transfer material 36 is cured by irradiating it with an energy ray such as an ultraviolet ray from the energy ray source 37. Next, the base body 11, which is integrated with the cured transfer material 36, is peeled off. This results in the film-shaped member 2 having multiple structures 12 formed on both sides of the base body 11, as shown in FIG. 15D. If necessary, the base layer 13 may be formed between the structures 12 and the base body 11.

The resin composition used as the transfer material 36 in this transfer step 2 can be the same as that used in the aforementioned transfer step 1.

In addition, a protective film can be attached to the surface of the film-shaped member 2 obtained in transfer step 1 or transfer step 2. This prevents the structures 12 of the film-shaped member 2 from being damaged in subsequent processes or during transportation.

### SHAPE-FORMING STEP

The film-shaped member 2 obtained as described above is cut into the required shape according to the protective equipment 21 to which the film-shaped member 2 is to be attached, and the tabs 20 are processed as appropriate. The tabs 20 can be processed using a numerically controlled cutting machine, a laser processing device, or a punching press. The use of a punching press is suitable because it allows the cutting to the specified shape and the physical processing of the tabs 20 to be performed in a single process.

### LAMINATING STEP

Next, the loop member 6 and the hook member 9 are fixed to each of the film-shaped members 2 in a specified pattern using a fixing material such as an adhesive or pressure-sensitive adhesive tape 10. Next, the film-shaped members 2 are laminated by engaging the loop member 6 and the hook member 9 of a pair of film-shaped members 2 that are to be laminated next to each other. This results in the film laminate 1. There is no particular limit to the number of layers of film-shaped members 2, and this can be set according to the intended use of the protective equipment. However, the more layers there are, the greater the impact on optical properties, so for applications where a clear field of vision is required, it is better to have fewer layers. In medical applications, there are cases where the entire film laminate 1 and protective equipment 21 must be disposable for hygienic reasons, so two or three layers are preferable.

### STERILIZATION STEP

Next, the film laminate 1 is sterilized. Sterilization can be carried out using gas sterilization with, e.g., ethylene oxide gas or radiation sterilization with, e.g., gamma rays. A gap is provided between each film-shaped member 2 of the film laminate 1. In addition, the loop member 6 and the hook member 9 are such that the loops 5 and hooks 8 erected on the base material 4, 7 are partially engaged without the base material 4, 7 being in close contact with each other, and there is an air permeable gap between the loop member 6 and the hook member 9. Therefore, the film laminate 1 can allow the sterilizing gas to pass through in a state in which the loop member 6 and the hook member 9 are engaged, i.e. in a state in which the film-shaped members 2 are laminated.

Therefore, it is possible to expose the entire exposed surface of all the film-shaped members 2 and the engagement portions of the loop member 6 and the hook member 9 to the sterilizing gas. Furthermore, in the film laminate 1, even if the film-shaped members 2 are formed using a material that does not allow the penetration of sterilizing gas such as ethylene oxide gas, it is still possible to sterilize the exposed entire surfaces sufficiently.

Sterilization is carried out after the film laminate 1 is formed, but before it is attached to the protective equipment 21 described below; however, sterilization may also be carried out after the film laminate 1 is attached to the protective equipment 21, or at both of these times.

During the use of the film laminate 1, when the topmost film-shaped member 2 becomes contaminated, by peeling off the contaminated topmost film-shaped member 2, the entire surface of the next film-shaped member 2 is exposed. The newly exposed film-shaped member 2, including the loop member 6 and the hook member 9, has been sterilized on all exposed surfaces, so the risk of infection to the patient, the examinee, and the medical personnel can be reduced.

In addition, the film-shaped member 2 can be peeled off simply by releasing the engagement between the loop member 6 and the hook member 9, so there is no risk of damaging the moth-eye structure formed on the film-shaped member 2. In this respect, in a laminated structure where the film-shaped members 2 are bonded together with a pressure-sensitive adhesive when the film-shaped member 2 is peeled off, there is a risk of mechanical damage such as damage to the interface between the pressure-sensitive adhesive layer and the film-shaped members 2 or cohesion failure between the pressure-sensitive adhesive layers, which may damage the moth-eye structure and have a negative impact on visibility, depending on the bonding strength. In addition, the scattering of adhesive residue and the generation of outgases from the pressure-sensitive adhesive layer are particularly undesirable in medical settings.

In a laminated structure in which the film-shaped members 2 are bonded to each other by welding, there is also a risk of damage to the moth-eye structure, which affects visibility, because the bonding between the film-shaped members 2 involves physical destruction when they are peeled off. In addition, the scattering of residue due to the destruction of the welding points is particularly undesirable in medical settings.

Since the film laminate 1 does not use pressure-sensitive adhesive and the film-shaped members 2 are not welded, there are no risks associated with them, so the film laminate 1 can be used safely. In addition, the film laminate 1 is composed of multiple film-shaped members 2 laminated together with air gaps between them, and the sides of the loop member 6 and the hook member 9 fixed to each film-shaped member 2 are not exposed to the outside, and the sterilized loop member 6 and hook member 9 are exposed. Therefore, the film laminate 1 can be exposed to sterilizing gas on the entire front and back surfaces of each film-shaped member 2, including the first and second engagement members, and only the sterilized portions of the newly revealed film-shaped members 2 are exposed when the film-shaped member 2 is peeled off.

### PROTECTIVE EQUIPMENT

The film laminate 1 is attached to the protective equipment 21. There is no particular restriction on the protective equipment 21, as long as it is used to ensure visibility by peeling off the film-shaped member 2; examples of the protective equipment 21 may include medical protective clothing (coveralls), medical face shields, medical eye shields, medical displays, helmet visors, protective glasses for painting, and chemical protective clothing used in times of disaster, among others.

The protective equipment 21 provided with the film laminate 1 has the film laminate 1 attached to the area corresponding to the face or eyes of the user wearing the protective equipment 21. If the surface of the film laminate 1 becomes contaminated during the use of the protective equipment 21, the topmost film-shaped member 2 can be peeled off to quickly restore visibility while avoiding contact with the contaminant. As the front surface and the back surface of each layer of the film-shaped member 2 in the film laminate 1, including the loop member 6 and the hook member 9, are sterilized, the sterilized surface always appears on the front surface, not only at the beginning of use but also after peeling. This reduces the risk of infection to the patient, the examinee, and other medical personnel in medical applications. In addition, since the back surface of the peeled-off film-shaped member 2 has also been sterilized, there is no risk of exposing the patient, examinee, and medical personnel to unsterilized areas when the film-shaped member 2 is peeled off or disposed of.

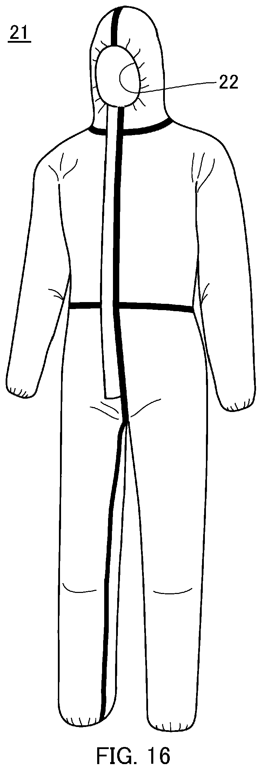

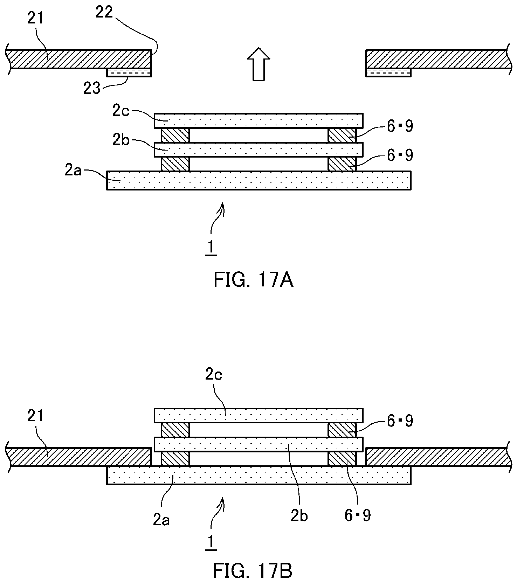

There is no particular restriction on the attaching method of the film laminate 1, and the method may be decided as appropriate according to the specifications of the protective equipment 21 to be used. FIG. 16 is an exterior perspective view illustrating a protective suit for medical use as an example of protective equipment 21. For example, as shown in FIG. 16, the protective equipment 21 has an opening 22 in a position corresponding to the face of the user, and the film laminate 1 is attached to this opening 22. **In** the film laminate 1 shown in FIG. 17A, three film-shaped members 2 are laminated; specifically, the bottommost film-shaped member 2a (i.e. the film-shaped member 2a that is positioned closest to the user's face), the intermediate film-shaped member 2b, and the topmost film-shaped member 2c are laminated via the loop members 6 and the hook members 9. The number of layers of the film-shaped members 2 that constitute the film laminate 1 according to the present technology is not limited to three.

The film-shaped members 2a, 2b and 2c are laminated with air gaps therebetween, and the loop member 6 and the hook member 9 are also permeable, so that sterilization treatment can be performed on the entire surface of the film laminate 1 exposed to the outside when the film laminate 1 is first used or when the film-shaped member 2 is peeled off. Furthermore, even if, when viewed from the side, there appears to be no apparent gap between one film-shaped member 2 and the other film-shaped member 2, among the film-shaped members 2a, 2b and 2c, these films are not in close contact with each other but only slightly touch each other, and the sterilizing gas can pass through the layers.

As shown in FIG. 17A, the bottommost film-shaped member 2a of the film laminate 1 is formed larger than the opening 22. In addition, an adhesive layer 23 using double-sided tape or the like is provided on the outer edge of the back side of the opening 22. The topmost film-shaped member 2c and the intermediate film-shaped member 2 of the film laminate 1 are inserted through the opening 22 from the back side of the protective equipment 21, and the bottommost film-shaped member 2a is adhered to the periphery of the opening 22 via the adhesive layer 23. This allows the film laminate 1 to be attached to the protective equipment 21, as shown in FIG. 17B.

It should be noted that the film-shaped members 2 are laminated by the loop member 6 or the hook member 9 provided on one side of one film-shaped member 2 engaging with the loop member 6 or the hook member 9 provided on the other side of the adjacent facing film-shaped member 2, and the side on which the loop member 6 is provided and the side on which the hook member 9 is provided are freely selectable. For convenience, the figures in this application are labeled "6 · 9" to indicate that the loop member 6 on one film-shaped member 2 is engaged with the hook member 9 on the other film-shaped member 2.

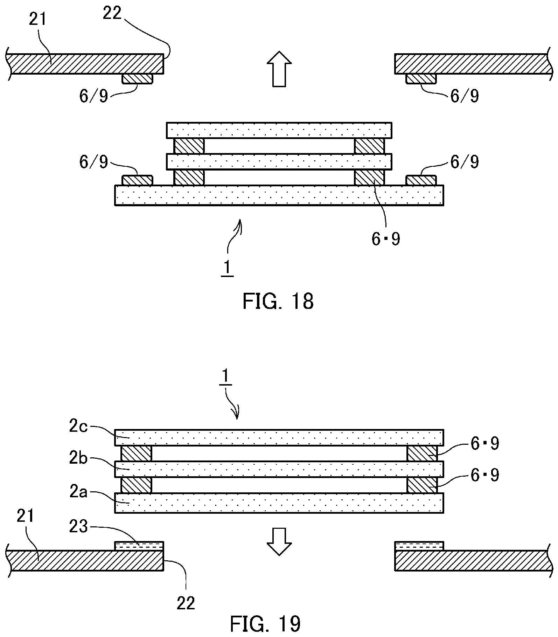

In addition, as shown in FIG. 18, the loop member 6 and the hook member 9 may be used in place of the adhesive layer 23. In the configuration shown in FIG. 18, either the loop member 6 or the hook member 9 is provided on the outer edge of the back side of the opening 22 and on the bottommost film-shaped member 2a. For convenience, the figures in this application are labeled "6 / 9" to indicate that one of the loop member 6 and the hook member 9 is provided. The other of the loop member 6 and the hook member 9 is provided at the facing position.

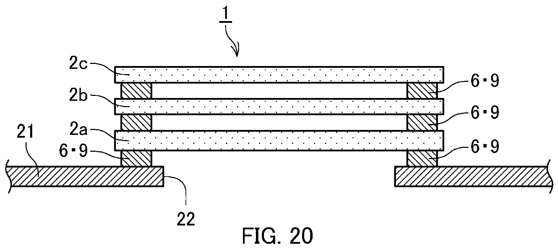

As shown in FIG. 19, the bottommost film-shaped member 2a may be attached to the front surface side of the protective equipment 21. In this case, the adhesive layer 23 using double-sided tape or the like is provided on the outer edge of the front surface side of the opening 22. The bottommost film-shaped member 2a of the film laminate 1 is then attached to the front surface side of the opening 22 via the adhesive layer 23. In the configuration shown in FIG. 19, the intermediate film-shaped member 2b and the topmost film-shaped member 2c having an area larger than the opening 22 can be used. In addition, in a configuration where the bottommost film-shaped member 2a is attached to the front surface side of the protective equipment 21, the loop member 6 and the hook member 9 may be used in place of the adhesive layer 23, as shown in FIG. 20.

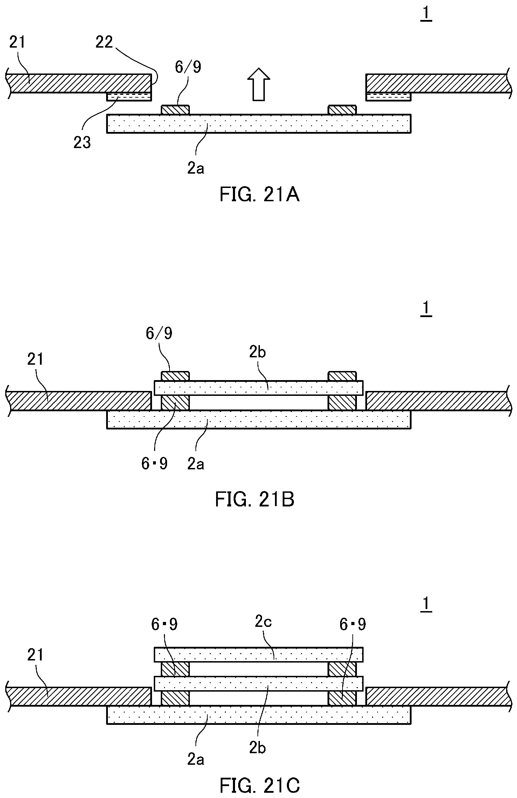

In addition, as shown in FIG. 21, the film laminate 1 and the protective equipment 21 provided with the film laminate 1 may be simultaneously completed by connecting the film-shaped member 2a to the opening 22 of the protective equipment 21 (FIG. 21A), laminating the intermediate film-shaped member 2b (FIG. 21B), and then laminating the topmost film-shaped member 2c (FIG. 21C).

### ANOTHER CONFIGURATION EXAMPLE 1 OF FILM LAMINATE

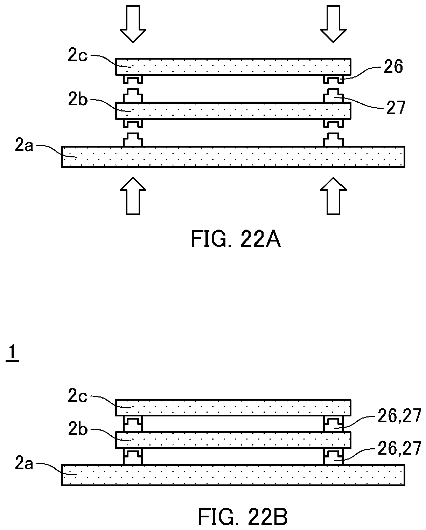

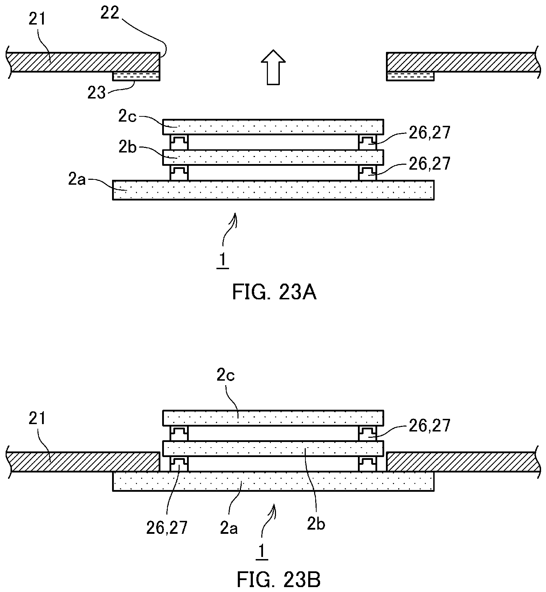

Next, another configuration example of the film laminate 1 will be explained. FIG. 22 is a cross-sectional view illustrating another configuration example of a film laminate according to the present technology, in which FIG. 22A shows the state before the film-shaped members 2 are laminated, and FIG. 22B shows the state after the film-shaped members 2 are laminated. FIG. 23 is a cross-sectional view illustrating the step of attaching a film laminate 25 shown in FIG. 22 to protective equipment, in which FIG. 23A shows the state before the film laminate 25 is attached, and FIG. 23B shows the state after the film laminate 25 is attached.

The film laminate 25 shown in FIG. 22 includes a first engagement member in the form of a recessed member 26 provided on one of the film-shaped members 2, and a second engagement member in the form of a protruding member 27 provided on the other film-shaped member 2, which releasably fits the recessed member 26. The first film-shaped member 2 and the second film-shaped member 2 are held and laminated with a gap therebetween by the fitting of the recessed member 26 and the protruding member 27.

The recessed member 26 and the protruding member 27 are partially fitted without being in close contact with each other, and there is a permeable gap in the fitting portion of the recessed member 26 and the protruding member 27. Therefore, the film laminate 25 allows sterilizing gas to pass between the recessed member 26 and the protruding member 27 when the recessed member 26 and the protruding member 27 are fitted, i.e. when the film-shaped members 2 are laminated. The film-shaped member 2 is peeled off by decoupling the fitting between the recessed member 26 and the protruding member 27. At this time, since the fitting portion of the permeable recessed member 26 and the protruding member 27 have been sterilized, the sterilized recessed member 26 and the protruding member 27 are exposed.

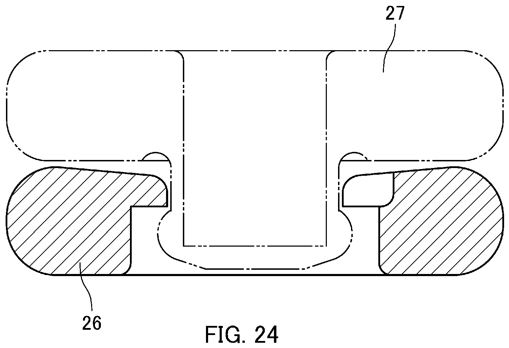

Examples of the snap buttons shown in FIG. 24 can be used as the releasably fitting recessed member 26 and protruding member 27. There is no particular restriction on the materials used for the recessed member 26 and protruding member 27, and e.g. plastic or metal parts can be used.

The recessed member 26 and the protruding member 27 are provided at facing positions when the film-shaped members 2 are laminated. In addition, the recessed member 26 and the protruding member 27 are fixed to the film-shaped member 2 using a fixing material 10 such as an adhesive or adhesive tape. The fixing strength of this fixing material 10 is higher than the engagement force of the recessed member 26 and the protruding member 27. Therefore, when the film-shaped member 2 is peeled off, the recessed member 26 and the protruding member 27 are not peeled off in the fitted state at the interface with one or the other film-shaped member 2, and the surface that has been sterilized can always be exposed. Also, when the film-shaped member 2 is peeled off, the fixing material 10 itself will not cause a cohesion failure between the fixed layers.

The recessed member 26 and the protruding member 27 are provided in such a position that the film-shaped member 2 can be stably held and laminated with a gap therebetween without obstructing the user's field of vision when attached to the protective equipment 21, and the film-shaped member 2 can be easily peeled off; for example, the recessed member 26 and the protruding member 27 may be provided on the outer edge of the film-shaped member 2. There is no particular restriction on the number of recessed members 26 and protruding members 27, as long as the film-shaped members 2 can be laminated and held in place stably.

As shown in FIG. 23, the film laminate 25 is attached to the protective equipment 21 by bonding the adhesive layer 23, which is provided on the outer edge of the back surface side of the opening 22, to the bottommost film-shaped member 2a, but the film laminate 25 may also be attached to the front surface side of the protective equipment 21. In addition, instead of the adhesive layer 23, the recessed member 26 and the protruding member 27 may be used as a means of attaching the film laminate 25 to the back or front side of the protective equipment 21.

### ANOTHER CONFIGURATION EXAMPLE 2 OF FILM LAMINATE

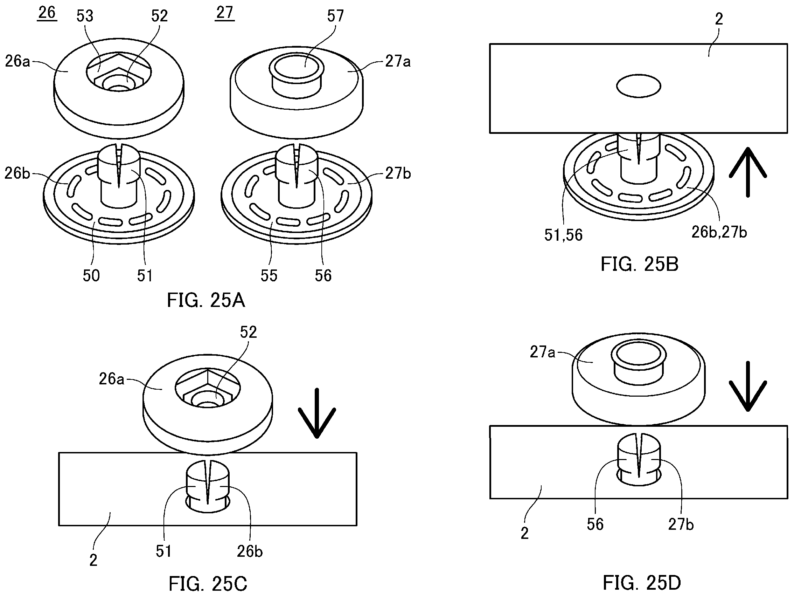

The recessed member 26 and the protruding member 27 may be fixed to the film-shaped member 2 by sandwiching the film-shaped member 2. The recessed member 26 shown in FIG. 25A includes a base 26b having a circular substrate 50 with a protrusion 51 erecting up from it, and a lid 26a having a fitting recess 53 formed around an insertion hole 52 through which the protrusion 51 is to be inserted. The lid 26a has the insertion hole 52 in the bottom of the fitting recess 53. The protrusion 51 of the base 26b is inserted into the hole opened in the film-shaped member 2 (FIG. 25B), and the tip of the protrusion 51 is inserted into the insertion hole 52 of the lid 26a from the opposite side (FIG. 25C). The tip of the protrusion 51 is slightly thicker than the insertion hole 52, and has a slit in the length direction, so it has flexibility in the radial direction. Therefore, the tip of the protrusion 51 that has passed through the insertion hole 52 of the lid 26a engages with the surroundings of the insertion hole 52, and this holds the film-shaped member 2 between the lid 26a and the base 26b.

The protruding member 27 shown in FIG. 25A includes a base 27b with a protrusion 56 erecting up on a substrate 55 and a lid 27a with a cylindrical fitting protrusion 57 formed thereon. The lid 27a has an insertion hole through which the protrusion 56 passes through the bottom of the fitting protrusion 57. The protrusion 56 of the base 27b is inserted through the hole opened in the film-shaped member 2 (FIG. 25B), and the tip of the protrusion 56 is inserted through the insertion hole in the lid 27a from the opposite side (FIG. 25D). The tip of the protrusion 56 has the same flexibility in the radial direction as with the protrusion 51. Therefore, the tip of the protrusion 56 that has passed through the insertion hole in the lid 27a engages with the periphery of the insertion hole, and this holds the film-shaped member 2 between the lid 27a and the base 27b.

The open end of the fitting recess 53 of the recessed member 26 has a projection that extends inward. On the other hand, the tip of the fitting protrusion 57 of the protruding member 27 has a projection that extends outward. When the fitting protrusion 57 is inserted into the fitting recess 53, the projections engage with each other, allowing the recessed member 26 and protruding member 27 to be fitted.

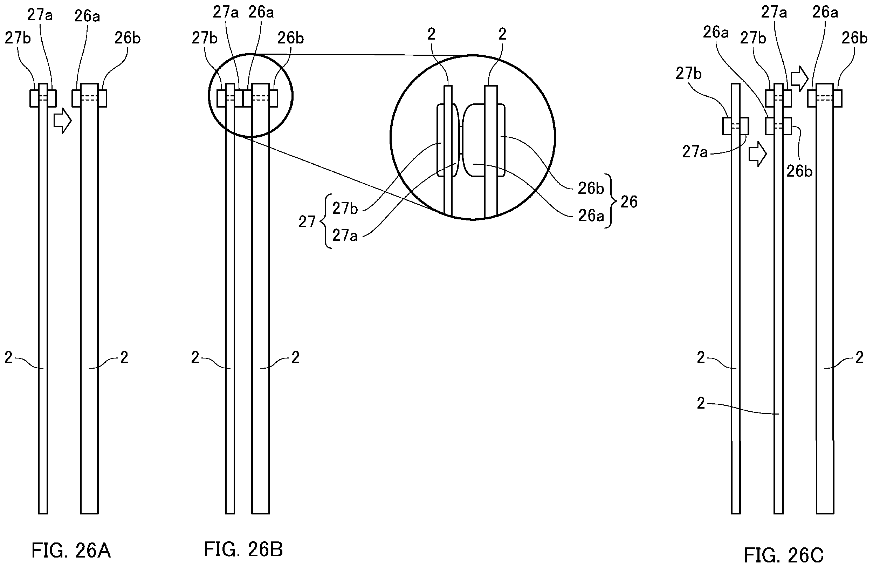

As shown in FIG. 26A, the film-shaped members 2 can be laminated by fixing the recessed member 26 to one side of the adjacent film-shaped member 2 and fixing the protruding member 27 to the other side, and then fitting the recessed member 26 and the protruding member 27. As shown in FIG. 26B, the fitting recess 53 of the recessed member 26 and the fitting protrusion 57 of the protruding member 27 are partially fitted without being in close contact, and there is also a permeable gap between the respective lids 26a, 27a of the recessed member 26 and the protruding member 27. Therefore, the film laminate 25 allows sterilizing gas to pass between the recessed member 26 and the protruding member 27 when the recessed member 26 and the protruding member 27 are fitted, i.e. when the film-shaped members 2 are laminated. The film-shaped member 2 is peeled off by decoupling the fitting recess 53 of the recessed member 26 and the fitting protrusion 57 of the protruding member 27. At this time, since the fitting portion of the permeable recessed member 26 and the protruding member 27 have been sterilized, the sterilized recessed member 26 and the protruding member 27 are exposed.

FIG. 26B shows a film laminate 1 made by laminating two layers of film-shaped members 2. FIG. 26C shows a film laminate 1 made by laminating three layers of film-shaped members 2. As shown, when laminating three or more layers of film-shaped members 2, the recessed member 26 and the protruding member 27 are fixed in such a manner that the fitting recess 53 or fitting protrusion 57 faces outward from both sides of the film-shaped member 2 constituting the intermediate layer.

### ANOTHER CONFIGURATION EXAMPLE 3 OF FILM LAMINATE

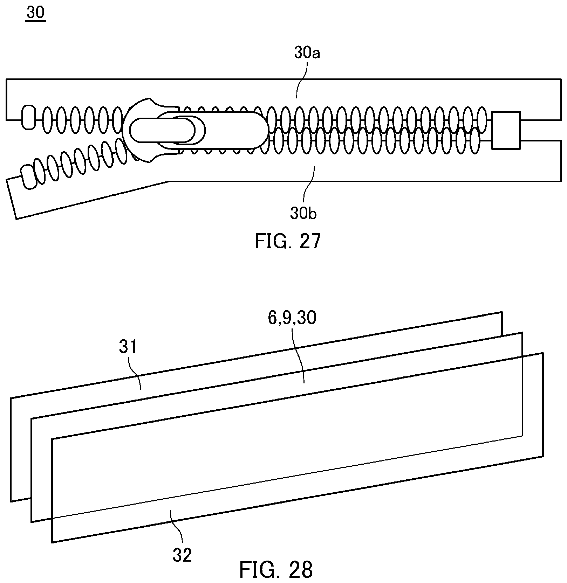

The first and second engagement members releasably engageable with each other may also be a zipper 30 such as that shown in FIG. 27. In this case, one half 30a of the zipper 30 is fixed along the side edge of one of the film-shaped members 2, and the other half 30b of the zipper 30 is fixed along the side edge of the other film-shaped member 2. The two film-shaped members 2 are laminated with a gap therebetween by closing the zipper. In addition, the two halves 30a and 30b of the zipper 30 are engaged without being in close contact, and there is a gap and air permeability at the engagement part of the two halves 30a and 30b.

Therefore, it is possible to pass the sterilizing gas in a state in which the zipper 30 is closed, i.e., the film-shaped members 2 are laminated. The film-shaped member 2 is peeled off by opening the zipper 30. At this time, the air-permeable zipper 30 has been sterilized so that the sterilized two halves 30a, 30b of the zipper 30 are exposed.

### EXAMPLES

Next, examples of the present technology will be explained. In the following, samples of the example of the film laminate according to the present technology were prepared by laminating the film-shaped members 2 using the loop member 6 and the hook member 9 as the first and second engagement members, respectively, and samples of the comparative example were prepared by laminating the film-shaped members 2 bonded with pressure-sensitive adhesive.

### First Examples

In first examples, the film-shaped members with different structures and laminating means were prepared, and the force for peeling (peel strength [N/25mm]) was measured and evaluated. In addition, the transmittance [%] and the haze [%]) of each film laminate sample was measured.

### PEEL STRENGTH

The film laminate samples used in the peel strength test were made by laminating a pair of rectangular film-shaped members (hereinafter referred to as "first film 31" and "second film 32"). The lamination means were the loop member 6 and the hook member 9 having the base materials 4 and 7 of the same size as the first film 31 and second film 32 or a pressure-sensitive adhesive film 30 corresponding to the entire surface (see FIG. 28).

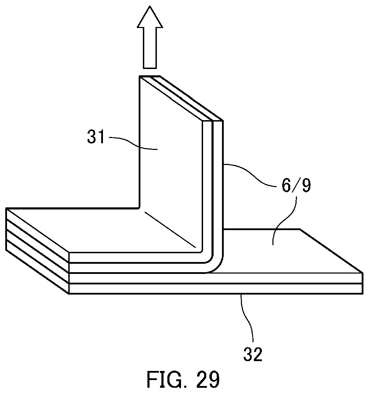

The peel strength [N/25mm] was measured using a 90° low-speed peeling test with a force gauge (DST-20N) made by IMADA (see FIG. 29). The measurement condition was set at a tensile speed of 200 mm/sec. The measurement sample size was 25 x 150 mm. The ease of peeling was evaluated by sensory evaluation of the ease of peeling when peeled in the TD direction and was evaluated in order of ease of peeling as follows: E (excellent), G (good), N (normal), and B (bad).

### TRANSMITTANCE AND HAZE

The transmittance [%] and haze [%] were determined using a haze meter (HM-150N) manufactured by MURAKAMI COLOR RESEARCH LABORATORY using the double beam method (JIS K 7361, JIS K 7136). A ϕ150mm integrating sphere was used. The measurement sample was a film laminate consisting of the first film and second film, each with a size of 50 x 50 mm, and with the two edges of each film laminated with the loop member 6 and the hook member 9 with a width of 12.5 mm, or with the pressure-sensitive adhesive film 30.

### GAP

The gap, i.e. the width of the void (mm), between the first and second films was measured. The gap was determined by subtracting the thickness of the first and second films from the thickness of the laminate sample measured with an M-type standard caliper N30 manufactured by Mitutoyo Corporation.

### Example 1

In Example 1, a first film 31 having a moth-eye structure formed on a polyethyleneterephthalate (PET) base material with a thickness of 188 µm and a second film 32 having a moth-eye structure formed on a PET base material with a thickness of 188 µm were laminated together using the loop member 6 and the hook member 9 to prepare a film laminate sample. In Example 1, the hook member 9 with mushroom-shaped hooks 5 (hereinafter referred to as "loop/hook 1") was used.

### Example 2

In Example 2, a first film 31 having a moth-eye structure formed on a PET base material with a thickness of 188 µm and a second film 32 having a moth-eye structure formed on a polycarbonate (PC) base material with a thickness of 150 µm were laminated together using the loop member 6 and the hook member 9 with the mushroom-shaped hooks 5 (loop/hook 1) to prepare a film laminate sample.

### Example 3

In Example 3, a first film 31 having a moth-eye structure formed on a PC base material with a thickness of 150 µm and a second film 32 having a moth-eye structure formed on a PC base material with a thickness of 150 µm were laminated together using the loop member 6 and the hook member 9 with the mushroom-shaped hooks 5 (loop/hook 1) to prepare a film laminate sample.

### (Example 4)

In Example 4, a first film 31 formed of a PET base material with a thickness of 188 µm and a second film 32 formed of a PET base material with a thickness of 188 µm were laminated together using the loop member 6 and the hook member 9 with the mushroom-shaped hooks 5 (loop/hook 1) to prepare a film laminate sample.

### Example 5

In Example 5, a first film 31 having a moth-eye structure formed on a PET base material with a thickness of 188 µm and a second film 32 having a moth-eye structure formed on a PET base material with a thickness of 188 µm were laminated together using the loop member 6 and the hook member 9 to prepare a film laminate sample. In Example 5, the hook member 9 with J-shaped hooks 5 (hereinafter referred to as "loop/hook 2") was used.

### Example 6

In Example 6, a first film 31 having a moth-eye structure formed on a PC base material with a thickness of 150 µm and a second film 32 having a moth-eye structure formed on a PC base material with a thickness of 150 µm were laminated together using the loop member 6 and the hook member 9 with the J-shaped hooks 5 (loop/hook 2) to prepare a film laminate sample.

### Comparative Example 1

In Comparative Example 1, a first film 31 having a moth-eye structure formed on a PET base material with a thickness of 188 µm and a second film 32 having a moth-eye structure formed on a PET base material with a thickness of 188 µm were laminated together using pressure-sensitive adhesive film 30 to paste the two edges of each film to prepare a film laminate sample.

**Table 1**

| | | Ex. 1 | Ex. 2 | Ex. 3 | Ex. 4 | Ex. 5 | Ex. 6 | Comp. 1 |
|---|---|---|---|---|---|---|---|---|
| first film (base material, thickness[µm]) | | PET188+moth-eye | PET188+moth-eye | PC150 + moth-eye | PET188 | PET188+moth-eye | PC150 + moth-eye | PET188+moth-eye |
| second film (base material, thickness[µm]) | | PET188+moth-eye | PC150+ moth-eye | PC150 + moth-eye | PET188 | PET188+moth-eye | PC150 + moth-eye | PET188+moth-eye |
| laminating means | | loop/hook 1 | loop/hook 1 | loop/hook 1 | loop/hook 1 | loop/hook 2 | loop/hook 2 | adhesive |
| peel strength[N/25mm] | Average | 0.71 | 0.88 | 1.6 | 0.5 | 3.09 | 2.76 | 0.61 |
| | Max | 2.12 | 0.89 | 3.23 | 0.89 | 9.01 | 8.66 | 0.69 |
| | Min | 0.23 | 0.25 | 0.76 | 0.25 | 0.64 | 0.38 | 0.58 |
| | variation *σ* | 0.37 | 0.12 | 0.41 | 0.34 | 1.75 | 1.46 | 0.04 |
| transmittance[%] | | 97.3 | 97.7 | 98.4 | 86.1 | 97 | 98.4 | 97.5 |
| haze[%] | | 0.3 | 0.4 | 0.5 | 1.4 | 0.4 | 0.5 | 0.3 |
| GAP[mm] | | 0.71 | 0.698 | 0.696 | 0.674 | 3.17 | 3.146 | 0.17 |
| ease of peeling | | E | E | E | E | N | N | G |

As shown in Table 1, the average peel strength of the film laminate samples for Examples 1 to 4, which used the loop/hook 1, was lower than that of the film laminate samples for Examples 5 and 6, which used the loop/hook 2. In addition, it was found that the film laminate samples for Examples 1 to 4 have excellent ease of peeling. The hook member 9 of the loop/hook 1 is flexible, and the mushroom shape of the hook 5 is believed to create this flexibility and contribute to the ease of peeling.

The average peel strength of the laminate samples in Comparative Example 1, which used a pressure-sensitive adhesive, was equivalent to that of the film laminate samples in Examples 1 to 4, which used the loop/hook 1, but the variation was smaller. In the evaluation of ease of peeling, the peel strength of Comparative Example 1 was felt to be heavier than that of Examples 1 to 4. In other words, it was found that the more variation there was in the peel strength, the easier it was to peel off.

Furthermore, when comparing Example 1 and Example 4, which were laminated using the same film base material (PET) and the loop/hook 1, Example 1 had better transmittance and haze. This shows that the effect of the moth-eye structure on visibility is high. It can also be seen that the transmittance is higher when the moth-eye structure is provided on the PC material than when it is provided on the PET material.

### Second Examples

In second examples, the film-shaped members with different structures and laminating means were prepared, and the peel strength (peel strength [N/25mm]) was measured and evaluated. Each film laminate sample was formed by laminating a first film 31 having a moth-eye structure formed on a PET base material with a thickness of 188 µm and a second film 32 having a moth-eye structure formed on a PET base material with a thickness of 188 µm. The loop member 6 and the hook member 9, or the pressure-sensitive adhesive film 30 were used as the laminating means. The sizes of the first and second films 31 and 32 were 25 x 150 mm. The measurement and evaluation conditions for the peel strength were the same as those for the first examples above.

### Example 1

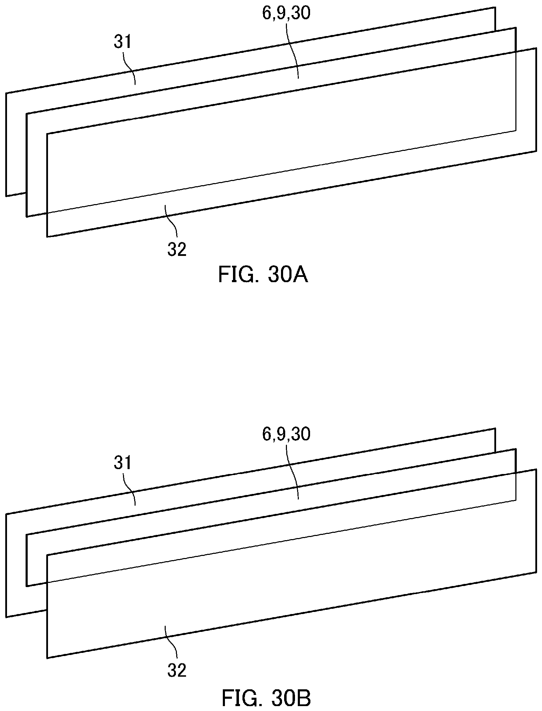

In Example 1, as shown in FIG. 30A, a film laminate sample was prepared using the loop/hook 1 with a base material dimension of 25 x 150 mm.

### Example 7

In Example 7, as shown in FIG. 30B, a film laminate sample was prepared using the loop/hook 1 with a base material dimension of 12.5 x 150 mm. The loop member 6 and the hook member 9 were pasted to the approximate center of the width of the first and second films.

### Example 5

As described above, in Example 5, a film laminate sample was prepared using the loop/hook 2 with a base material dimension of 25 x 150 mm.

### Example 8

In Example 8, a film laminate sample was prepared using the loop/hook 2 with a base material dimension of 12.5 x 150 mm. The loop member 6 and the hook member 9 were pasted to the approximate center of the width of the first and second films.

### Comparative Example 1

As described above, in Comparative Example 1, a film laminate sample was prepared by laminating the first film 31 and second film 32 by pasting the entire surface with the same-sized pressure-sensitive adhesive film 30.

### Comparative Example 2

In Comparative Example 2, the first film 31 and the second film 32 were laminated together using an adhesive film with a width of 12.5 mm, which is half the width of the first and second films to prepare a film laminate sample. The pressure-sensitive adhesive films were pasted to the approximate center of the width of the first and second films 31 and 32.

**Table 2**

| | | Ex. 1 | Ex. 7 | Ex. 5 | Ex. 8 | Comp. 1 | Comp. 2 |
|---|---|---|---|---|---|---|---|
| first film (base material, thickness[µm]) | | PET188+Moth | PET188+Moth | PET188+Moth | PET188+Moth | PET188+Moth | PET188+Moth |
| second film (base material, thickness[*µ* m]) | | PET188+Moth | PET188+Moth | PET188+Moth | PET188+Moth | PET188+Moth | PET188+Moth |
| laminating means | | loop/hook 1 | loop/hook 1 | loop/hook 2 | loop/hook 2 | adhesive | adhesive |
| widh of laminating means | | 25mm | 12.5 mm | 25mm | 12.5mm | 25mm | 12.5 mm |
| peel strength[N/25mm] | Average | 0.71 | 0.45 | 3.09 | 1.64 | 0.61 | 0.26 |
| | Max | 2.12 | 1.05 | 9.01 | 5.08 | 0.69 | 0.32 |
| | Min | 0.23 | 0.04 | 0.64 | 0.03 | 0.58 | 0.17 |
| | variation *σ* | 0.37 | 0.2 | 1.75 | 1.1 | 0.04 | 0.03 |
| ease of peeling | | E | E | N | N | G | G |

As shown in Table 2, regardless of the laminating means, the peel strength is reduced by approximately 40 to 60% by halving the width. In other words, it is possible to control the peel strength by adjusting the width of the laminating means, and it is possible to set the optimal peel strength according to the application.

### Third Examples

In the third example, film laminate samples with different laminating means and different fixing positions for laminating film-shaped members were prepared, and the samples were exposed to sterilizing gas to verify the effect of sterilization. The sterilizing gas used was ethylene oxide gas (EOG). The film-shaped members used were rectangular films with a size of 100 x 120 mm. Stickers (sterilization label EO-L manufactured by Nippon Oil & Energy Technology Co., Ltd.) that reveal the word "sterilized" when they react with EOG were pasted to the center of the film-shaped members.

The sterilization conditions were as follows.
Temperature: 50°C
Humidity: 50% RH
Exposure time: 8 hours
Chamber pressure: 100 kPa, decompression pressure: -85 kPa
Flushing: 5 times

The results were evaluated as follows: if the word "sterilized" appeared, the result was marked as G (good: sterilization was effective); if the word "sterilized" did not appear, the result was marked as B (bad: sterilization was not effective).

### Example 9

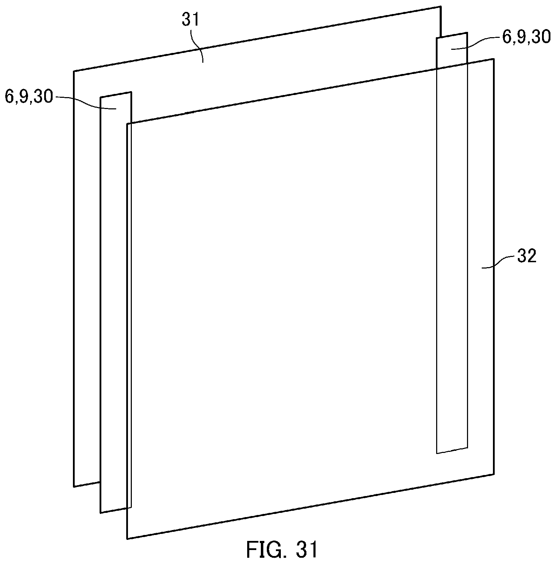

In Example 9, a first film 31 formed of a PET base material with a thickness of 188 µm and a second film 32 formed of a PET base material with a thickness of 188 µm were laminated together using the loop member 6 and the hook member 9 with the mushroom-shaped hooks 5 (loop/hook 1) to prepare a film laminate sample. As shown in FIG. 31, in Example 9, the loop/hook 1 was provided at both ends of the longitudinal direction of the first and second films.

### Example 10

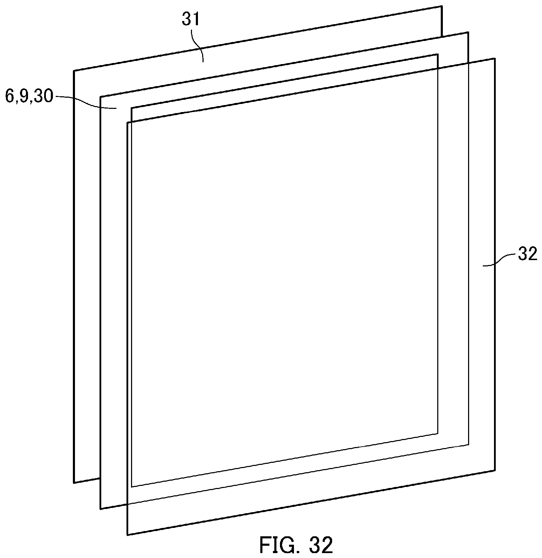

Example 10 was the same as Example 9, except that the loop/hook 1 was provided to surround the entire circumference of the side edges of the first and second films, as shown in FIG. 32.

### Example 11

Example 11 was the same as Example 9, except that the loop/hoop 2 was used as the laminating means.

### Comparative Example 3

Comparative Example 3 was the same as Example 10, except that adhesive film 30 was used as the laminating means.

| | Ex. 9 | Ex. 10 | Ex. 11 | Comp. 3 |
|---|---|---|---|---|
| first film (base material, thickness[*µ* m]) | PET188+Moth | PET188+Moth | PET188+Moth | PET188+Moth |
| second film (base material, thickness[µm]) | PET188+Moth | PET188+Moth | PET188+Moth | PET188+Moth |
| laminating means | loop/hook 1 | loop/hook 1 | loop/hook 2 | adhesive |
| fixing method | both ends | four edges | both ends | four edges |
| sterilization | G | G | G | B |

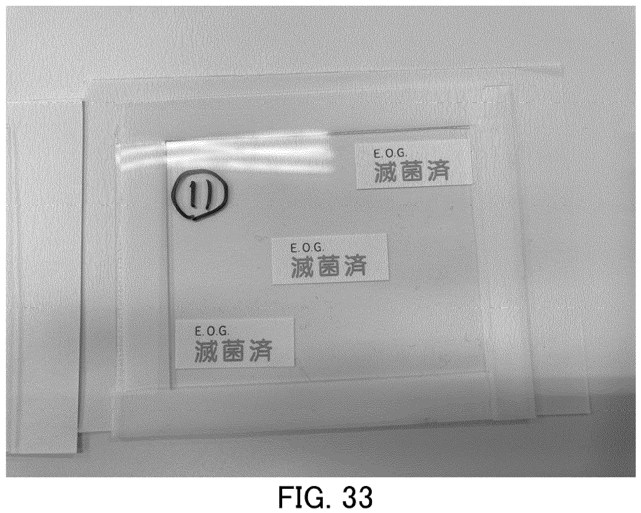

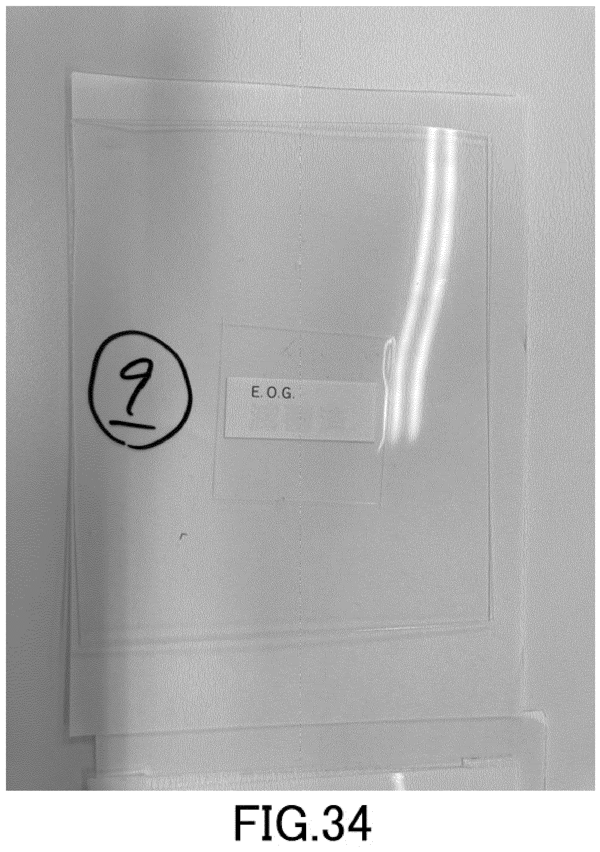

As shown in Table 3, even in Example 10, where loop/hook 1 were provided to surround all four sides of the first and second films, the word "sterilized" appeared on the sticker provided in the center, confirming the effectiveness of the sterilization (FIG. 33). This shows that the loop/hook 1 is permeable, and that it is possible to sterilize the gap in the center of the film-shaped member, as well as the loop/hook 1 itself. On the other hand, in Comparative Example 3, where the pressure-sensitive adhesive was applied to surround all four sides of the first and second films, the word "sterilized" did not appear on the sticker pasted in the center, indicating that the portion could not be sterilized (FIG. 34). Therefore, when the first and second films are peeled off, the unsterilized area is exposed.

In Example 9 where loop/hook 1 was provided at both ends of the first and second films and in Example 11 where loop/hook 2 was provided at both ends of the first and second films, the effectiveness of sterilization was confirmed.

### REFERENCE SIGNS LIST

1: film laminate, 2: film-shaped member, 4: base material, 5: loop, 6: loop member, 7: base material, 8: hook, 9: hook member, 10: fixing material, 11: base body, 12: structure, 13: base layer, 20 tab, 21 protective equipment, 23 adhesive layer, 25 film laminate, 26 recessed member, 27 protruding member, 30 pressure-sensitive adhesive, 31 first film, 32 second film, 34 hook member, 35 pressure-sensitive adhesive, 36 transfer material, 37 energy ray source, 41 roll master, 42 structure

## Claims

1. A film laminate, comprising:
a plurality of film-shaped members laminated in a separable manner; and
a first engagement member that is fixed to one of a pair of the film-shaped members and a second engagement member that is fixed to the other of the pair of film-shaped members, wherein
the first and second engagement members are releasably engaged with each other to laminate the film-shaped members with a gap therebetween, and
the first and second engagement members include engagement portions having air permeability.

2. The film laminate according to claim 1, wherein
the first engagement member is a loop member having multiple loops erected on one surface of a base material, and
the second engagement member is a hook member having multiple hooks erected on one surface of a base material that are removably engaged with the loops, and
the film-shaped members are laminated with a gap by engaging the loop member and the hook member.

3. The film laminate according to claim 2, wherein the peel strength of the loop member and the hook member that cause the film-shaped members of a relatively upper layer to be laminated with each other is lower than the peel strength of the loop member and the hook member that cause the film-shaped members of a relatively lower layer to be laminated with each other.

4. The film laminate according to any one of claims 1 to 3, wherein the engagement force of the first engagement member and the second engagement member is lower than the fixing force between the first engagement member and the film-shaped member, and lower than the fixing force between the second engagement member and the film-shaped member.

5. The film laminate according to claim 1, wherein
the first engagement member is a recessed member,
the second engagement member is a protruding member that releasably fits the recessed member, and
the film-shaped members are laminated with a gap therebetween by the fitting of the recessed member and the protruding member.

6. The film laminate according to claim 5, wherein the peel strength of the recessed member and the protruding member that laminate the film-shaped members of the upper layer is relatively lower than the peel strength of the recessed member and the protruding member that laminate the film-shaped members of the lower layer.

7. The film laminate according to any one of claims 1 to 3, 5, and 6, wherein the first and second engagement members are respectively provided on an outer edge of the film-shaped member.

8. The film laminate according to any one of claims 1 to 3, 5, and 6, wherein the first and the second engagement members are provided so as to surround the outer edge of the film-shaped member.

9. The film laminate according to any one of claims 1 to 3, 5, and 6, wherein externally exposed surfaces of each of the film-shaped members and the first and second engagement members are sterilized.

10. The film laminate according to any one of claims 1 to 3, 5, and 6, wherein the film-shaped member includes multiple structures provided on at least one surface of a flexible transparent base material at a pitch smaller than the wavelength of visible light.

11. A method for manufacturing a film laminate, comprising:
a step of forming film-shaped members;
a step of forming a first engagement member and a second engagement member that are releasably engaged with each other;
a step of fixing the first engagement member to one film-shaped member of a pair of film-shaped members at a position facing the other film-shaped member of the pair of film-shaped members, and fixing the second engagement member to the other film-shaped member of the pair of film-shaped members at a position facing the one film-shaped member of the pair of film-shaped members; and
a step of laminating the pair of film-shaped members with a gap therebetween by engaging the first and second engagement members.

12. The method for manufacturing a film laminate according to claim 11, further comprising a step of sterilizing the film laminate with multiple film-shaped members laminated.

13. Protective equipment in which a film laminate is attached to a part corresponding to the face or eyes of a user, wherein
the film laminate is the film laminate according to any one of claims 1 to 3, 5, and 6.

14. The protective equipment according to claim 13, wherein the protective equipment is protective clothing, a face shield, an eye shield, or a helmet.

15. A method for manufacturing protective equipment in which a film laminate is attached to a part corresponding to the face or eyes of a user, comprising:
a step of forming a film laminate; and
a step of attaching the film laminate to protective equipment, wherein
the film laminate is the film laminate according to any one of claims 1 to 3, 5, and 6.
